# EUROPEAN PATENT APPLICATION

(11) **EP 2 218 435 A1**
(43) Date of publication of application: **18.08.2010**
(21) Application number: 10001323.4
(22) Date of filing: 09.02.2010
(51) Int. Cl.: A61K 6/00, A61K 6/083, C07C 409/00, C08F 4/28, C08F 4/40, C08L 33/04

(54) **Initiators in two components, and polymerizable composition using the same**

(30) Priority: 12.02.2009 JP 2009029599
(71) Applicant: GC Corporation, Itabashi-ku Tokyo 174-8585 (JP)
(72) Inventor: Yarimizu, Hideki, Tokyo 174-8585 (JP); Tokui, Hideki, Tokyo 174-8585 (JP); Nakaseko, Hisashi, Tokyo 174-8585 (JP)
(74) Representative: Müller-Boré & Partner Patentanwälte

(57) **Abstract**

To provide initiators in two components having no discoloring after polymerization and excellent polymerization-curability under the existence of water, and provide a polymerizable composition using the initiators in two components. The initiators in two components are for polymerizing and curing a (meth)acrylate compound, and the initiator includes (a) a peroxide being compatible or soluble with water, (b) an aromatic sulfinic acid compound, (c) a tert-amine compound, and (d) water. The polymerizable composition uses the initiators in two components. Particularly, preferably, the polymerizable composition includes a first component and a second component. The first component comprising (a) a peroxide being compatible or soluble with water, and a (meth)acrylate compound, and the second component includes (b) an aromatic sulfinic acid compound, (c) a tert-amine compound, and (d) water.

## Description

The present invention relates to initiators in two components of a polymerizable composition. The initiators are included in two parts of liquids or pastes, or powder and liquid containing (meth) acrylate compounds, which are used by mixing the components.

For example, Japanese Patent Application Laid-Open No. 62-246514 discusses a conventionally used method for polymerizing a polymerizable composition using achemical polymerization catalyst at room temperature. The polymerizable composition includes polymerizable (meth)acrylates or oligomers and prepolymers of these (meth)acrylates. As for the chemical polymerization catalysts, the combination of an organic peroxide and an aromatic tert-amine has been used. This method can control a polymerization start time and increase preservation stability of the composition before polymerization by adjusting amounts of the organic peroxide and the aromatic tert-amine which are blended in the polymerizable composition, and together using a polymerization inhibitor. However, the method has a problem that the aromatic tert-amine in the initiator discolors a cured product after polymerization. Further, the conventional polymerization initiator obtained by combining an organic peroxide and an aromatic tert-amine cannot provide (meth)acrylate compounds with adequate polymerizability under a watery oral condition.

On the other hand, Japanese Patent Application Laid-Open No. 10-114615 discusses a technique in which an initiator using an oxidization of trialkylborane is used to obtain the (meth)acrylate compound which can cure even when being used at the watery condition. However, since trialkylborane remarkably, easily oxidizes more than the aromatic tert-amine, this technique has a fault that it is hard to include trialkylborane in a composition before polymerization. Thus, since trialkylborane is preserved in a separate vessel from the (meth)acrylate compound, trialkylborane must be added to the composition from the vessel whenever using. Therefore, the preservation and operation are annoying.

The present invention is directed to initiators in two components capable of preventing a polymerizable composition after polymerization from discoloring and having excellent curability even under a watery oral condition. In addition, the present invention is directed to a polymerizable composition consisting of two components.

The present inventors carried out earnest works to solve the aforementioned problems and, as a result, they found out the followings to complete the present invention. In the present invention, an organic peroxide which is compatible or soluble with water is used as an peroxide. An aromatic sulfinic acid compound is used as a reducing material. A tert-amine compound is used as an accelerator. Water is blended to dissolve and activate the peroxide. As a result of this, the present invention can obtain initiators in two components capable of preventing a polymerizable composition after polymerization from discoloring and having excellent polymerization-curability even under a watery oral condition. In addition, the present invention can thus obtain a polymerizable composition using the initiators in two components.

According to a formulation of the present invention, the present invention relates to initiators in two components for polymerizing a (meth)acrylate compound. The initiators include (a) a peroxide which is compatible or soluble with water, (b) an aromatic sulfinic acid compound, (c) a tert-amine compound, and (d) water.

A polymerizable composition using the initiators in two components according to the present invention includes first and second embodiments. The first embodiment is a polymerizable composition including a first component and a second component. The first component includes (a) a peroxide which is compatible or soluble with water, and a (meth) acrylate compound. The second component includes (b) an aromatic sulfinic acid compound, (c) a tert-amine compound, and (d) water. The second embodiment is a polymerizable composition including a powder component and a liquid component. The powder component includes (a) a peroxide which is compatible or soluble with water, (b) an aromatic sulfinic acid compound, and a reactive filler. The liquid component includes (c) a tert-amine compound, (d) water, and a (meth)acrylate compound having an acid group.

The initiators in two components according to the present invention and the polymerizable composition using the initiators in two components do not discolor after polymerizing, and can have excellent polymerization curability of the composition even under the existence of water.

As for (a) a peroxide which is compatible or soluble with water in the initiators in two components according to the present invention, potassium peroxodisulfate, sodium peroxodisulfate, ammonium peroxodisulfate, tert-butyl hydroperoxide, stearoyl peroxide, succinic acid peroxide, a hydrogen peroxide-polyvinylpyrrolidone complex, or the like can be used. These can be used independently or by mixing two or more kinds. Particularly, potassium peroxodisulfate and tert-butyl hydroperoxide are preferable from viewpoints of solubility to water and excellent polymerizing property.

As for (b) an aromatic sulfinic acid compound, a conventionally used compound can be used. That is, the aromatic sulfinic acid compound could be sodium p-toluenesulfinate, lithium p-toluenesulfinate, benzenesulfinic acid, sodium benzenesulfinate, p-toluenesulfonyl chloride, p-toluenesulfonyl fluoride, o-toluenesulfonyl isocyanate, p-toluenesulfonyl hydrazide, p-toluenesulfonamide, p-toluenesulfonyl imidazole, p-toluenesulfonyl cyanide, 2-(p-toluenesulfonyl) acetophenone, p-toluenesulfonyl-N-diethylamide, α-N, α-toluenesulfonyl-N-arginine, α-N, p-toluenesulfonyl-L-arginine methyl ester, p-toluenesulfonyl methyl isocyanate, p-toluenesulfonyl-N-methyl-N-nitrosamide, N-(p-toluenesulfonyl)-L-phenylalanine, N-p-toluenesulfonyl-L-phenylalanyl chloride, p-toluenesulfonyl acetonitrile, 2-(p-toluenesulfonyl) acetophenone, toluene-3, 4-disulfonyl chloride, benzenesulfonamide, benzenesulfohydroxamic acid, benzenesulfonyl chloride, benzenesulfonyl isocyanate, benzenesulfonyl anilide, benzenesulfonchloramide sodium, benzenesulfondichloramide, benzenesulfonylhydrazide, benzenesulfonyl-N-methylamide, 2-phenylsulfonylacetophenone, diaminodiphenyl sulfone, 4, 4'-sulfonyldiphenol, sulfapyridine, sulfaaerosol, sulfamethysol, ethylbenzenesulfonyl chloride, nitrobenzenesulfonyl chloride, nitrobenzenesulfonyl fluoride, or the like. In addition, the organic aromatic compounds can be hydrates.

(c) a tert-amine compound could be either an aromatic tert-amine or an aliphatic tert-amine. More particularly, N, N-dimethyl-p-toluidine, N, N-diethyl-p-toluidine, N, N-dimethylaniline, N, N-bis(2-hydroxyethyl)-p-toluidine, N, N-dimethylaminoethyl methacrylate, triethanolamine, methyl 4-dimethylaminobenzoate, ethyl 4-dimethylaminobenzoate, isoamyl 4-(dimethylamino)benzoate, triethylamine, N-ethyldiethanolamine, triethanolamine, or the like can be used. These can be used independently or by mixing two or more kinds.

(d) Water is blended in (a) a peroxide which is compatible or soluble with water, and to activate the peroxide.

As for a (meth)acrylate compound, a conventionally used (meth)acrylate monomer, oligomer and prepolymer can be used without restriction. One example is a compound having unsaturated double bond, e.g., unsaturated polyester or the like can be used. More concretely, the compound could be methyl (meth)acrylate, ethyl (meth)acrylate, isopropyl (meth)acrylate, 2-hydroxyethyl (meth)acrylate, 3-hydroxypropyl (meth)acrylate, 2-hydroxypropyl (meth)acrylate, 2-hydroxy-1, 3-di(meth)acryloxy propane, n-butyl (meth)acrylate, isobutyl (meth)acrylate, tetrahydrofurfryl (meth)acrylate, glycidyl (meth)acrylate, 2-methoxyethyl (meth)acrylate, 2-ethylhexyl (meth)acrylate, benzyl (meth)acrylate, phenyl (meth)acrylate, phenoxyethyl (meth)acrylate, 2, 2-bis(meth)acryloxyphenyl propane, 2, 2-bis[4-(2-hydroxy-3-(meth)acryloxypropoxy)phenyl] propane, 2, 2-bis(4-(meth)acryloxydiethoxyphenyl) propane, 2, 2-bis(4-(meth)acryloxypolyethoxyphenyl) propane, ethylene glycol di(meth)acrylate, diethylene glycol di(meth)acrylate, triethylene glycol di(meth)acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, (meth)acrylate having urethane bond in a molecule, e.g., di-2-(meth)acryloxyethyl-2, 2, 4-trimethylhexamethylene dicarbamate, or the like. These (meth)acrylates and acrylates can be used independently or by mixing two or more kinds, or can be used as an oligomer or prepolymer.

In the present invention, a (meth)acrylate compound having an acid group can be selected and used as the (meth)acrylate compound, corresponding to the embodiments described below. The (meth)acrylate compound having an acid group had an effect for giving adhesive property to the polymerizable composition to adhere to tooth structure and dental restorative materials which are ceramics such as zirconia and alumina, or an alloy including noble metals. The (meth)acrylate compound having an acid group is preferably (meth)acrylate having a phosphate group or a carboxyl group. Thus, a (meth)acrylate compound having one or plural phosphate groups or carboxyl groups in one molecule can be used. Since the phosphate group has acidity stronger than the carboxyl group, the phosphate group has a high effect for dissolving a smear layer of a tooth surface and for tooth demineralization. Particularly, the phosphate group can exercise an effect for improving adhesive property to enamel. The (meth)acrylate compound having the phosphate group is a polymerizable monomer having one or plural phosphate groups in one molecule. For example, the (meth)acrylate compound having a phosphate group could be 2-(meth)acryloyloxyethyldihydrogen phosphate, bis(meth)acryloxyethyl phosphate, bis[2-(meth)acryloyloxyethyl] hydrogen phosphate, 2-(meth)acryloyloxyethylphenylhydrogen phosphate, acid phosoxyethyl (meth)acrylate, 6-(meth)acryloyloxyhexyldihydrogen phosphate, 6-(meth)acryloyloxyhexylphenylhydrogen phosphate, 10-(meth)acryloyloxydecyldihydrogen phosphate, 1, 3-di(meth)acryloylpropane-2-dihydrogen phosphate, 1, 3-di(meth)acryloylpropane-2-phenylhydrogen phosphate, bis[5-{2-(meth)acryloyloxyethoxycarbonyl} heptyl] hydrogen phosphate, a reaction product of anhydric phosphoric acid and a 6-hexanolide addition polymer of 2-hydroxyethyl(meth)acrylate, or the like. Particularly, 10-(meth)acryloyloxydecyldihydrogen phosphate is preferable because of having excellent adhesive property and self-stability. These (meth)acrylate compounds having the phosphate group can be used independently or by mixing two or more kinds.

The (meth)acrylate compound having the carboxyl group could be 4-(meth)acryloxyethyltrimellitic acid, 4-(meth)acryloxyethyltrimellitic acid anhydride, 4-(meth)acryloxydecyltrimellitic acid, 4-(meth)acryloxydecyltrimellitic acid anhydride, 11-(meth)acryloyloxy-1, 1-undecanedicarboxylic acid, 1, 4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, or the like. Particularly, 4-(meth)acryloxyethyltrimellitic acid and 4-(meth)acryloxyethyltrimellitic acid anhydride are preferable because of having an excellent adhesive property. These (meth)acrylate compounds having the carboxyl group can be used independently or by mixing two or more kinds.

As for a first embodiment, the polymerizable composition using the initiators in two components according to the present invention includes a polymerizable composition consisting of a two-liquid type or two-paste type initiators in two components which includes first component and second component. The first component includes (a) a peroxide which is compatible or soluble with water, and a (meth) acrylate compound. The second component includes (b) an aromatic sulfinic acid compound, (c) a tert-amine compound, and (d) water. The polymerizable composition can be used for a dental resin cement. The polymerizable composition is not adversely influenced by water in the mouth, and can thus have an excellent adhesive property and excellent preservability.

A polymerizable composition obtained by integrating conventional cement components with the polymerizable composition for the first embodiment can be also effectively used as a dental cement. That is, the polymerizable composition is a two-liquid type or two-paste type which consists of a first component and a second component. The first component includes (a) a peroxide which is compatible or soluble with water, a powder component including a reactive filler, and the (meth)acrylate compound. The second component includes (b) an aromatic sulfinic acid compound, (c) a tert-amine compound, (d) water, and a polymer of α-β unsaturated monocarboxylic acid or α-β unsaturated dicarboxylic acid. A nonreactive filler can be blended with one or both of the first component and the second component if needed.

As for a second embodiment, the polymerizable composition using the initiators in two components according to the present invention is a powder type polymerizable composition including a powder component and a liquid component. The powder component includes (a) a peroxide which is compatible or soluble with water, (b) an aromatic sulfinic acid compound, and a powder component including a reactive filler. The liquid component includes (c) a tert-amine compound, (d) water, and a (meth)acrylate compound. This polymerizable composition includes the powder component and liquid component, and is not adversely influenced by water content in the mouth like the first embodiment. Thus, the polymerizable composition can have excellent adhesive property and preservability. In the second embodiment, a nonreactive filler can be blended with one or both of the powder component and the liquid component if needed in order to utilize features of a dental cement. Further, in addition to or instead of the (meth)acrylate compound having the acid group, a polymer of α-β unsaturated monocarboxylic acid or α-β unsaturated dicarboxylic acid can be used.

The polymer of α-β unsaturated monocarboxylic acid or α-β unsaturated dicarboxylic acid used in the present invention is a copolymer or a homopolymer including one or more kinds selected from acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid. The polymer preferably has a weight average molecular weight of 5,000 to 40,000 without an ethylenically unsaturated double bond. If the weight average molecular weight is less than 5, 000, the strength of a cured product decreases easily, and the adhesive strength to the tooth structure tends to decrease. If the weight average molecular weight is more than 40,000, the viscosity when kneading is too high, and the kneading thus tends to be hard.

The nonreactive filler is blended for increasing strength of the polymerizable composition. If the reactive filler further indicates a cement reaction, excellent characteristics of conventional dental cement can be used. The nonreactive filler could be powders such as anhydrous silicic acid, glasses, such as barium glass, alumina glass, potassium glass, and the like, calcium phosphate, feldspar, fumed silica, aluminum silicate, calcium silicate, magnesium carbonate, hydrous silicic acid, hydrous calcium silicate, hydrous aluminum silicate, quartz, or the like. In order to chemically bond with (meth) acrylate, the nonreactive filler can be treated with a silane coupling agent, such as γ-methacryloxypropyltrimethoxysilane, vinyltrichlorosilane, vinyltriethoxysilane, vinyltrimethoxysilane, vinyltriacetoxysilane, vinyltri (methoxyethoxy) silane, or the like. Further, a pre-polymerized filler produced by previously mixing the aforementioned nonreactive filler with a (meth)acrylate compound, polymerizing the mixture, and pulverizing the cured product, can be used as well. Particularly, anhydrous silicic acid, hydrous silicic acid, hydrous calcium silicate, and hydrous aluminum silicate have an effect for preventing the polymerizable composition before polymerization from gelling even when being stored for a long period of time.

The reactive filler makes a cement reaction with a polymer of α-β unsaturated monocarboxylic acid or α-β unsaturated dicarboxylic acid and an acid group of the acid (meth) acrylate with water existence. The reactive filler could be fluoroaluminosilicate glass or synthetic zeolite.

The polymerizable composition according to the present invention can contain a photopolymerization initiator, a thickener, a pigment, a stabilizer, and/or an antimicrobial agent.

### [Examples]

The present invention will be described in detail below using examples, but the present invention is not restricted in the examples.

Polymerizable compositions were produced at blending ratios illustrated in Tables 1 and 2. The two-paste type polymerizable composition is used by mixing the first paste and the second paste at the weight ratio of 1:1. The powder and liquid type polymerizable composition is used by mixing the power and the liquid at the weight ratio of 2:1.

Brevity codes in the tables are as follows. UDMA: Di-2-methacryloxyethyl-2, 2, 4-trimethylhexamethylene dicarbamate
TEGDMA: Triethylene glycol dimethacrylate
HEMA: 2-hydroxyethyl methacrylate
BPO: Benzoyl peroxide
4-META: 4-methacryloxyethyltrimellitic acid anhydride
MDP: 2-methacryloyloxyethyldihydrogen phosphate Quartz powder: Power having a particle diameter of 5µm or less (produced by Tatsumori Co., Ltd.)
Aerosil (fumed silica) powders: Powders having primary particle diameters from 10 to 20 nm (produced by Nippon Aerosil Co., Ltd.)
Aerosil (aluminum oxide) powder: Powders having primary particle diameters from 10 to 20 nm (produced by Nippon Aerosil Co., Ltd.)

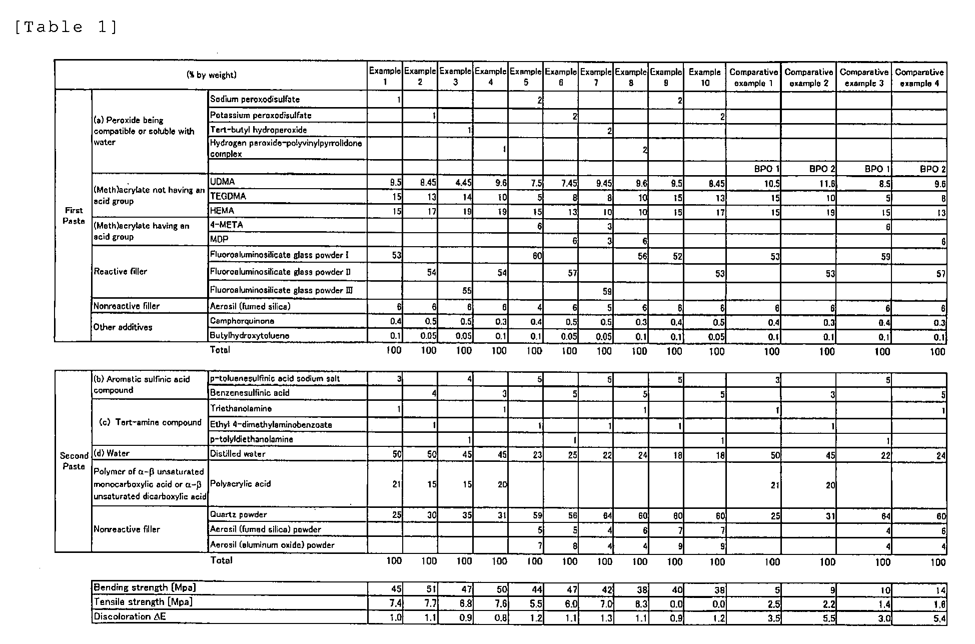

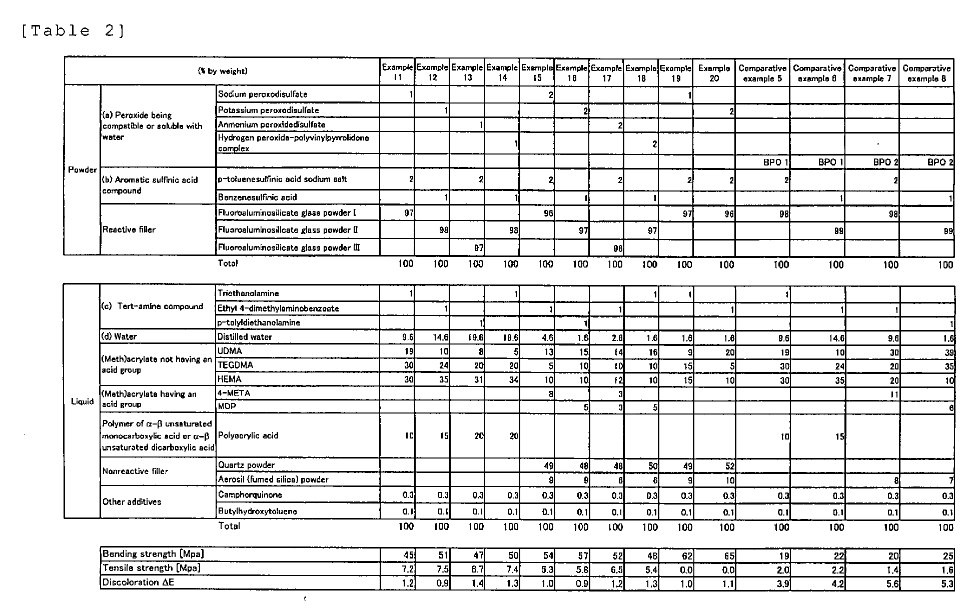

The blending ratios of fluoroaluminosilicate glass powders I, II and III were shown in Table 3.

**[Table 3]**

| | Fluoroaluminosilicate glass powders | | |
|---|---|---|---|
| | I | II | III |
| Aluminum oxide (g) | 21 | 23 | 22 |
| Anhydrous silicic acid (g) | 44 | 41 | 43 |
| Calcium fluoride (g) | 12 | 10 | 12 |
| Calcium phosphate (g) | 14 | 13 | 15 |
| Strontium carbonate (g) | 9 | 13 | 8 |

Each of the fluoroaluminosilicate glass powders I and III was produced by fully mixing raw materials, putting the mixture in a high temperature electric furnace at 1200°C for 5 hours so as to melt a glass, cooling the melted glass, pulverizing the glass for 10 hours using a ball mill, and sieving the pulverized glass with 200 meshes (ASTM). The fluoroaluminosilicate glass powder II was produced by a process similar to that of the fluoroaluminosilicate glass powders I and III except the glass was heated at 1100°C to melt.

### <Discoloring testing method>

According to the color tone stability of JIS T6514:2005 (dental composite resin for filling) 4.4, a sample was soaked in distilled water at 37°C for 7 days. Then, a color change (ΔE) of the sample before and after soaking was measured. The color was measured using SPECTROMETER CM-3610d produced by MINOLTA Co. Ltd. The lower ΔE indicates the less color change. These results were shown in Tables 1 and 2.

### <Tensile adhesive testing method>

The adhesive strength was evaluated under the existence of water. An extracted tooth of bovine was ground with a water-resistant grinding paper of #600, and an area of adhering surface was regulated to have a diameter of 3 mm by a plastic tape. The kneaded polymerizable composition.was applied to a stainless steel rod for a tensile jig, and was pressed to the area-regulated bovine tooth. The specimen was stored for 1 hour in a thermostat at 37°C and humidity of 100%, and then were soaked in distilled water at 37°C. After 24 hours from the start of kneading, a tensile test was performed at the cross head speed of 1 mm/lmin. These results were shown in Tables 1 and 2.

### <Bending testing method>

According to the bending test of JIS T6609-2:2005 (Dentistry - water-based cement - Part II: Resin-modified cements) 5. 12, a bending test was performed. These results were shown in Tables 1 and 2.

## Claims

1. Initiators in two components for polymerizing and curing a (meth)acrylate compound, the initiators comprising:
(a) a peroxide being compatible or soluble with water;
(b) an aromatic sulfinic acid compound;
(c) a tert-amine compound; and
(d) water.

2. A polymerizable composition using the initiators in two components according to claim 1, the polymerizable composition comprising:
a first component comprising (a) a peroxide being compatible or soluble with water, and a (meth) acrylate compound; and
a second component comprising (b) an aromatic sulfinic acid compound, (c) a tert-amine compound, and (d) water.

3. A polymerizable composition using the initiators in two components according to claim 1, the polymerizable composition comprising:
a powder component comprising (a) a peroxide being compatible or soluble with water, (b) an aromatic sulfinic acid compound, and a reactive filler; and a liquid component comprising (c) a tert-amine compound, (d) water, and a (meth)acrylate compound having an acid group.
